# EUROPÄISCHE PATENTSCHRIFT

(11) **EP 0 844 856 B1**
(45) Veröffentlichungstag und Bekanntmachung des Hinweises auf die Patenterteilung: **17.10.2001**
(21) Anmeldenummer: 96927612.0
(22) Anmeldetag: 26.07.1996
(51) Int. Cl.: A61F 2/44, A61F 2/46, A61B 19/00, A61B 17/88

(54) **ZWISCHENWIRBELIMPLANTAT UND INSTRUMENTE ZUM IMPLANTIEREN EINES ZWISCHENWIRBELIMPLANTATS**
INTERVERTEBRAL IMPLANT AND INSTRUMENTS FOR IMPLANTING AN INTERVERTEBRAL IMPLANT
IMPLANT INTERVERTEBRAL ET INSTRUMENTS POUR PLACER L'IMPLANT INTERVERTEBRAL

(30) Priorität: 11.08.1995 DE 19529605
(43) Veröffentlichungstag der Anmeldung: 03.06.1998
(73) Patentinhaber: Zientek, Bernhard, 73630 Remshalden (DE)
(72) Erfinder: Zientek, Bernhard, 73630 Remshalden (DE)
(74) Vertreter: Dreiss, Fuhlendorf, Steimle & Becker
(86) Internationale Anmeldenummer: EP9603306
(87) Internationale Veröffentlichungsnummer: WO9706753

(56) Entgegenhaltungen:
- EP-A- 0 637 439
- EP-A- 0 646 366
- EP-A- 0 664 994
- DE-A- 3 505 567
- DE-C- 4 323 956
- DE-C- 4 416 605
- FR-A- 2 717 068

## Beschreibung

Die Erfindung betrifft ein Zwischenwirbelimplantat mit einem etwa zylinderförmigen Hohlkörper, der zwei Enden, vier Seitenflächen sowie eine oder mehrere Öffnungen aufweist und mit zwei einander gegenüberliegenden aufspreizbaren Bereichen versehen ist, die zur Fixierung von Wirbelkörpern mit Fixierelementen versehen sind, an ihren einen Enden miteinander verbunden und an ihren anderen freien Enden durch Aufweitelemente aufspreizbar sind, und bei dem ein Ende des Zwischenwirbelimplantats eine mit einem Innengewinde versehene Öffnung aufweist.

Ein derartiges Zwischenwirbelkörperimplantat ist aus der EP 0 664 994 A1 (= US 5,554,191 A) bekannt. Zum Aufweiten sind zwei Bauteile erforderlich, nämlich eine zylindrische Aufweitrolle und eine Schraube, die von der Aufweitrolle in einen konischen Spalt zwischen den freien Enden der aufspreizbaren Bereiche gezogen wird. Die Schraube wird nicht in derselben Richtung in das Implantat eingesetzt, von der her sie zur Aufweitung der aufspreizbaren Bereiche eingeschraubt werden muss. Der Operateur kann also bei der Spreizung die freien Enden nicht beobachten. Es sind ferner drei Bauteile, nämlich der Hohlkörper, die Schraube und die Aufweitrolle vorgesehen. Ähnliches gilt für die DE 43 23 956 C1.

Bei der EP 0 637 439 A1 ist die Bauweise dadurch aufwendig, dass in dem eingesetzten Käfig auf jeder Seite vier bewegliche Aufspreizelemente eingesetzt und angelenkt sind, die durch zwei auf einer Schraube mittels Gewinde geführte konische Elemente auseinandergedrückt werden.

Allgemein gilt für das Einsetzen von Zwischenwirbelimplantaten Folgendes:
Zwischen den einzelnen Wirbelkörpern der Wirbelsäule befinden sich die Zwischenwirbelscheiben, die unter dem Begriff "Bandscheibe" besser bekannt sind. Die Bandscheiben bestehen aus einem bindegewebigen und knorpeligen äußeren Ring und einem inneren Gallertkern und dienen als elastischer Puffer zwischen den Wirbelkörpern. Der innere Gallertkern kann sich jedoch verlagern oder durch Risse im äußeren Ring austreten. Diese Krankheit ist als Bandscheibenvorfall bekannt. Bei schweren Fällen ist eine Nukleotomie oder Diskotomie erforderlich, d. h. die Bandscheibe muss entfernt werden. Anschließend müssen die Wirbelkörper fusioniert werden, d. h. der Abstand zwischen ihnen muss durch geeignete Maßnahmen aufrechterhalten werden, weil sie sonst in die Lücke, die vorher die Bandscheibe ausgefüllt hatte, nachrutschen würden. Dadurch käme es zu Stauchungen in diesem Bereich der Wirbelsäule; das Rückenmark würde komprimiert und in der Nachbarschaft der betroffenen Wirbelkörper gedehnt. Die Folge wären Schmerzen und neurologische Ausfälle. Dies erfolgt durch Zwischenwirbelimplantate.

Ein weiteres bekanntes Zwischenwirbelimplantat (P.L.I.F., posterolateral interbody fusion), sieht vor, dass käfigartige Hohlkörper (intersomatic cage) zwischen die Wirbelkörper gesetzt werden. Dazu wird in dem vorher von der Bandscheibe eingenommenen Bereich durch Aufrauhen der benachbarten Wirbelkörper bzw. Entfernen ihrer Knochenhaut und der Substantia Corticalis ein Raum geschaffen, in den der Hohlkörper eingetrieben wird. Nachteilig daran ist, daß bei diesem Verfahren immer eine zusätzliche Stabilisierung notwendig ist, z.B. eine interne Fixation durch 4 mit 2 Stäben verbundene Pedikelschrauben. Trotzdem können die Hohlkörper verrutschen, weil sie nur allmählich einwachsen. Das liegt daran, daß die Substantia Corticalis, die für das Knochenwachstum mitverantwortlich ist, in Mitleidenschaft gezogen ist. Insbesondere können die Implantate nach rückwärts weggedrückt werden, solange sie noch nicht fest eingewachsen sind.

Eine andere im Stand der Technik bekannte Möglichkeit ist die Implantation schraubbarer Hohlzylinder für die lumbale Wirbelkörperfusion. Bei diesen Implantaten handelt es sich um Hohlzylinder mit einem Außengewinde. Das bedeutet, daß in die dem Bandscheibenraum benachbarten Wirbelkörper ein Gewinde eingeschnitten werden muß. In dieses Gewinde werden dann die Hohlzylinder eingeschraubt. Bei diesem Verfahren können die Implantate zwar nicht mehr verrutschen, aber es ist extrem aufwendig, was ebenfalls die Gefahr von Komplikationen birgt. Die notwendige Verletzung der Knochenhaut und der Substantia Corticalis der Wirbelkörper wird ebenfalls als Nachteil empfunden, da diese Strukturen hart und fest sind und normalerweise stabilisierend wirken. Durch die Verletzung der Substantia Corticalis wird das Knochenwachstum und somit die Fusion verlangsamt (vgl. DE 35 05 567 A1).

Ein Nachteil, der den beiden bekannten Zwischenwirbelimplantaten gemein ist, sind ihre festen Maße. Der aufrechtzuerhaltende Abstand zwischen den betroffenen Wirbelkörpern ist jedoch bei jedem Patienten anders. Man braucht also zahlreiche Ausführungen mit verschiedenen Maßen. Trotzdem läßt es sich nicht vermeiden, daß die Zwischenwirbelimplantate nicht optimal sitzen, z.B. zu fest oder locker sitzen.

Aufgabe der Erfindung ist es daher, ein verstellbares Zwischenwirbelimplantat der o.g. Art bereitzustellen, mit dem die soeben beschriebenen Nachteile vermieden und auf möglichst einfache Weise eine schnelle und dauerhafte Fusion von Wirbelkörpern ermöglicht wird.

Die Lösung besteht darin, daß das Aufweitelement eine Schraube ist, die in die mit dem Innengewinde versehene Öffnung einschraubbar ist, und dass die Schraube mit ihrem Ende oder ihrem Kopf beim Einschrauben die freien Enden der aufspreizbaren Bereiche auseinanderdrückt.

Das erfindungsgemäße Zwischenwirbelimplantat weist Fixierelemente auf, die es gegen Lageveränderungen an den Wirbelkörpern sichern. Sie verankern das Zwischenwirbelimplantat an den Wirbelkörpern. Das Aufweiten kann in verschiedenem Maß erfolgen, so daß man variable Maße zur Verfügung hat, d.h. der Durchmesser des Zwischenwirbelimplantats und damit der Abstand der Wirbelkörper voneinander einstellbar ist. Damit hat man immer die richtige Größe, insbesondere den richtigen Durchmesser für jeden Patienten zur Verfügung. Ferner sitzt das Zwischenwirbelimplantat immer genau richtig, nämlich nicht zu fest und nicht zu locker. Der Spielraum beträgt ca. 1 bis 2 mm. Man braucht wesentlich weniger Zwischenwirbelimplantate verschiedener Maße für die variierenden Wirbelkörperabstände der Patienten. Die Weitungselemente dienen auch dazu, die Fixierelemente in "Einsetzstellung" zurückzuziehen, so daß der Hohlkörper ohne Hindernis zwischen die Wirbelkörper eingetrieben werden kann. Erst danach erfolgt das Aufweiten und damit die Herbeiführung der Festlegung der Fixierelemente an den Wirbelkörpern.

Das Aufweiten des Zwischenwirbelimplantats kann auf verschiedene Weise erfolgen. Eine Möglichkeit ist, im Hohlkörper ausgeschnittene Aufspreizzungen vorzusehen, die nach außen biegbar sind und an ihrer Außenfläche Fixierelemente aufweisen. Eine andere Möglichkeit besteht darin, daß der Hohlkörper selbst einen Schlitz mit einer gewissen Breite aufweist und dadurch in zwei Hälften geteilt ist, die sich zusammenpressen bzw. aufweiten lassen. In diesem Fall ist der Spielraum nicht durch den Durchmesser des Hohlkörpers selbst begrenzt.

Besonders vorteilhaft ist es, das erfindungsgemäße Zwischenwirbelimplantat in einer etwas balligen oder bauchigen Form zu gestalten. Damit ist es den Konturen des durch die Flächen der Wirbelkörper gebildeten, vorher von der Bandscheibe ausgefüllten Raumes in besonderer Weise angepaßt.

Das Aufweiten des erfindungsgemäßen Zwischenwirbelimplantats erfolgt bevorzugt in zwei Schritten. Da die aufweitbaren Bereiche zunächst zusammengezogen sind, wird es in einem ersten Schritt vorgeweitet, bis die Fixierelemente die Wirbelkörper berühren. In einem zweiten Schritt wird es dann endgültig geweitet und damit an den Wirbelkörper fixiert. Die Fixierelemente weisen vorteilhafterweise einen Absatz auf, in dem ein Rückholinstrument eingreifen kann, so daß sie nach dem Aufweiten ggf. wieder eingezogen werden können.

Die für die Implantation notwendigen Instrumente umfassen einen Implantathalter, mit dem das erfindungsgemäße Zwischenwirbelimplantat bis zu seiner endgültigen Fixierung leicht und sicher geführt werden kann. Dieser Implantathalter ist vorzugsweise hohl und kann andere Instrumente wie z.B. einen Distraktor zum Vorweiten oder ein Einsetzinstrument zum Einführen des Stellelements in sich aufnehmen. Damit ist jederzeit eine Kontrolle über Lage und Sitz des Zwischenwirbelimplantats während des Einsetzvorgangs gewährleistet.

Weitere vorteilhafte Weiterbildungen ergeben sich aus den Unteransprüchen.

Ausführungsbeispiele der vorliegenden Erfindung werden im folgenden anhand der beigefügten Zeichnungen näher beschrieben. Es zeigen:
- Figur 1: eine perspektivische Ansicht eines ersten Ausführungsbeispiels;
- Figur 2: eine Seitenansicht des in Figur 1 gezeigten Ausführungsbeispiels;
- Figur 3: eine Ansicht in Richtung III-III in Figur 2;
- Figur 4: einen Längsschnitt durch das in Figur 1 gezeigte Ausführungsbeispiel; dabei zeigt die Figur die obere Hälfte den aufgespreizten Zustand und die untere Hälfte den noch nicht aufgespreizten Einsetzzustand;
- Figuren 5 bis 8: ein zweites, dem ersten funktionsgleiches Ausführungsbeispiel entsprechend den Figuren 1 bis 4;
- Figur 9: eine schematische Darstellung zweier implantierter Ausführungsbeispiele nach Figur 5;
- Figuren 10 bis 12: drei Instrumente für die Implantation der in den Figuren 1 und 5 gezeigten Ausführungsbeispiele;
- Figur 13: ein Ausführungsbeispiel eines erfindungsgemäßen Implantathalters für die in den Figuren 1 und 5 gezeigten Implantate;
- Figur 14: ein Ausführungsbeispiel eines Distraktors;
- Figur 15: eine Darstellung des in Figur 14 gezeigten Distraktors in dem in Figur 13 gezeigten Implantathalter;
- Figuren 16 und 17: zwei weitere Instrumente;
- Figuren 18 und 19: Ausschnittvergrößerungen der Bereiche XVIII in Figur 15 und XV in Figur 16; die Figuren zeigen in der oberen Hälfte das Implantat mit Instrument vor der Betätigung, in der unteren Hälfte mit Instrument bzw. Element nach der Betätigung;
- Figur 20: eine Ausschnittvergrößerung des Bereichs XX in Figur 17; die Figur zeigt in der oberen Hälfte das Implantat nach der Betätigung, in der unteren Hälfte mit Instrument vor der Betätigung;
- Figur 21: ein weiteres Instrument zur Implantation;
- Figur 22: eine Draufsicht auf ein drittes Ausführungsbeispiel eines erfindungsgemäßen Zwischenwirbelimplantats;
- Figur 23: einen Schnitt entlang der Linie XXIII-XXIII in Figur 22 im Einsetzzustand;
- Figur 24: eine Ansicht in Richtung der Pfeile XXIV-XXIV in Figur 23;
- Figur 25: eine Darstellung wie Figur 23, jedoch in aufgespreiztem Zustand;
- Figur 26: eine Ansicht in Richtung der Pfeile XXVI-XXVI in Figur 25 in aufgespreiztem Zustand, ohne Schraube 77;
- Figuren 27 bis 31: Darstellungen eines dem dritten Ausführungsbeispiel funktionsgleichen vierten Ausführungsbeispiel entsprechend den Figuren 22 bis 26;
- Figur 32: ein Einsetzinstrument (Impaktor) für die Ausführungsbeispiele nach den Figuren 22 bis 31;
- Figuren 33 bis 34: Details der Aufnahme des Ausführungsbeispiels nach den Figuren 27 bis 31, entsprechend dem Ausschnitt XXXIII in Figur 32 (Figur 34 ist im Vergleich zu Figur 33 um 90° gedreht);
- Figur 35: eine Ansicht in Richtung der Pfeile XXXV-XXXV in Figur 34;
- Figur 36: eine schematische Darstellung eines Instruments zur Messung des Abstandes zwischen zwei Wirbelkörpern;
- Figur 37: eine Vergrößerung des Ausschnitts XXXVII in Figur 36.

Die nachfolgend beschriebenen Ausführungsbeispiele sind Teile aus Titan oder ggf. auch aus Carbon. Titan ist gewebeverträglich und besitzt ein hohes Elastizitätsmodul, so daß es ein hervorragend geeignetes Material für die erfindungsgemäßen Zwischenwirbelimplantate darstellt.

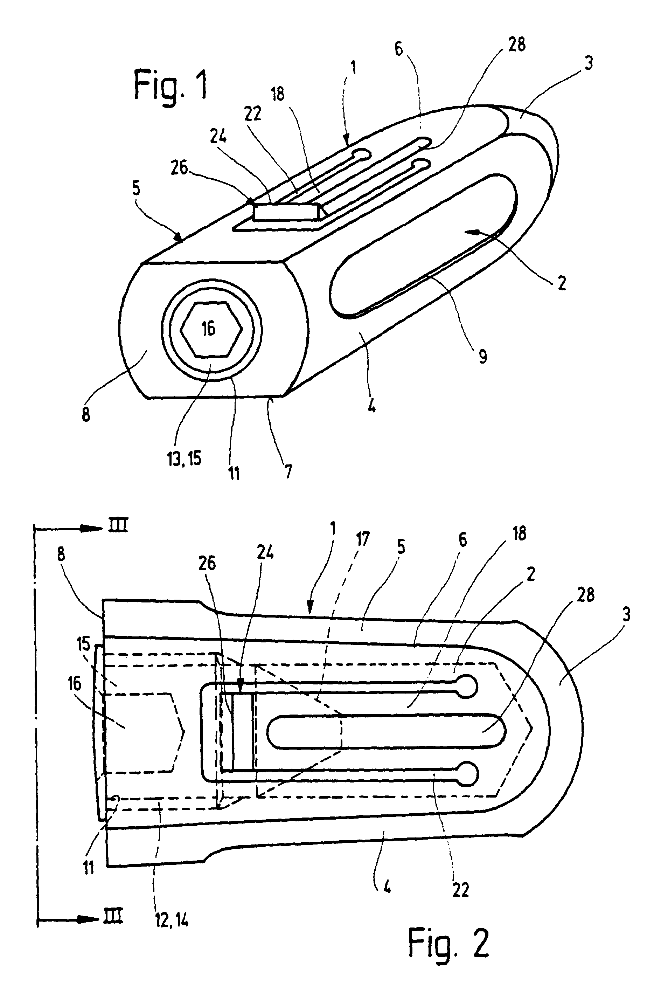

Die in den Figuren 1 bis 4 bzw. 5 bis 8 dargestellten Zwischenwirbelimplantate sind funktionsgleich und auch in wesentlichen Teilen baugleich. In den Figuren sind gleiche Teile mit denselben Bezugszeichen versehen.

Die Zwischenwirbelimplantate sind etwa 25 mm lang, 11 bis 14 mm breit und 9 bis 13 mm hoch. Sie weisen einen Hohlkörper 1 auf, der einen Hohlraum 2 umschließt. Der Hohlkörper 1 ist etwa zylinderförmig und mit einer Kuppe 3, vier Seitenflächen 4, 5, 6, 7 und eine Rückfläche 8 versehen. In die Seitenflächen 4, 5 sind ovale Öffnungen 9, 10 eingelassen, die sich über etwa die Hälfte bis zwei Drittel der Seitenflächen 4, 5 erstrecken. An der Rückfläche 8 befindet sich eine etwa mittig angeordnete Bohrung 11 mit einem Innengewinde 12, in das eine Schraube 13 mit einem Außengewinde 14 eingedreht ist. Der Kopf 15 der Schraube 13 weist einen Innensechskant 16 auf. Die Schraube bildet das eingangs erwähnte Aufweitelement.

In den beiden Seitenflächen 6, 7 befinden sich etwa rechteckige, ca. 4 mm breite Aufspreizzungen 18, 19, jeweils begrenzt durch einen U-förmigen Schlitz 22, 23. An dem freien Ende jeder Aufspreizzunge 18, 19 ist je eine Krampe 24, 25 einstückig angeformt. Die Krampen 24, 25 sind im Querschnitt dreieckig, derart, daß die freien Kanten 26, 27 nach außen ragen. Die freien Kanten 26, 27 sind scharf ausgebildet, z.B. angeschliffen. Die Krampen bilden die eingangs genannten Fixierelemente.

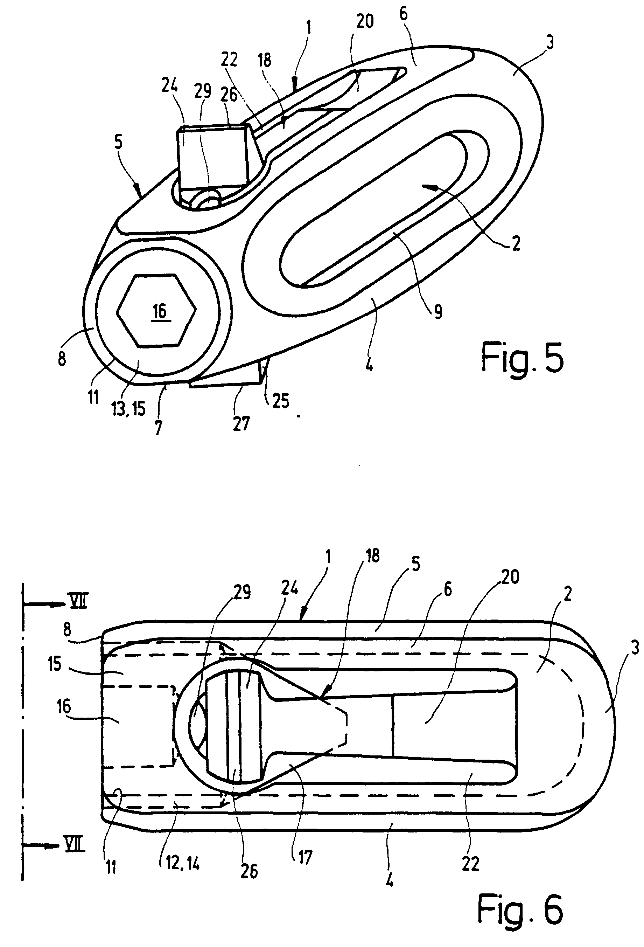

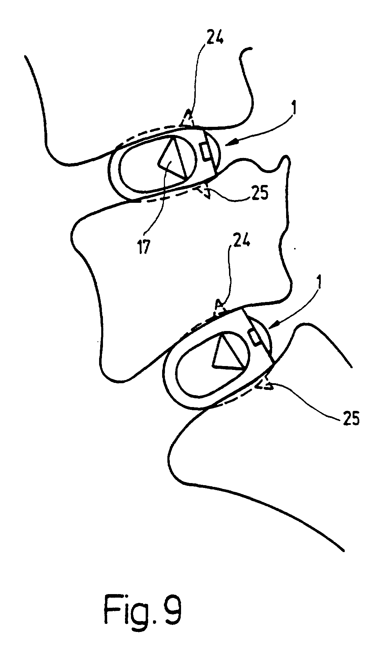

Die in den Figuren 1 und 5 dargestellten Ausführungsbeispiele weisen bei gleicher Funktion strukturelle Unterschiede auf. Das Ausführungsbeispiel in Figur 1 weist zwei flache Seitenflächen 6, 7 und zwei gewölbte Seitenflächen 4, 5 auf, während das zweite Ausführungsbeispiel in Figur 5 vorteilhafterweise eine insgesamt gewölbte, "ballige" oder "bauchige" Kontur hat und im Querschnitt angenähert quadratisch ist. Der Vorteil dieser Kontur ist in Figur 9 schematisch dargestellt. Die Flächen der Wirbelkörper, die den Hohlraum umschließen, in dem sich vorher die Bandscheibe befand, haben eine leicht konkave Kontur. Damit korrespondiert die eher konvexe Kontur des Implantats derart, daß es großflächig an den Wirbeln anliegt und sich gewissermaßen in den Hohlraum "einschmiegt".

Die Aufspreizzungen 18, 19 des ersten Ausführungsbeispiels (Figur 1) weisen ferner Langlöcher 28 auf, damit das Knochengewebe schneller den Hohlraum 2 durchdringen kann. Auf die Langlöcher 28 wurde beim zweiten Ausführungsbeispiel (Figur 5) verzichtet. Die Aufspreizzungen 18, 19 sind stattdessen schmaler und mit gebogenen Federelementen 20, 21 versehen, die ihnen eine größere Elastizität verleihen. Die Aufspreizzungen 18, 19 des zweiten Ausführungsbeispiels weisen ferner einen Absatz 29 auf, in den in aufgespreiztem Zustand ein Rückholinstrument eingesetzt werden kann, um bei Abstützung des Rückholinstruments an der Schulter des Gewindes 12 für die Schraube 13 die Aufspreizzungen 18, 19 wieder in den Hohlraum 2 einzuziehen (siehe dazu weiter unten). Der Absatz 29 ist rund ausgebildet, damit das Rückholinstrument nicht nach links oder rechts abrutschen kann.

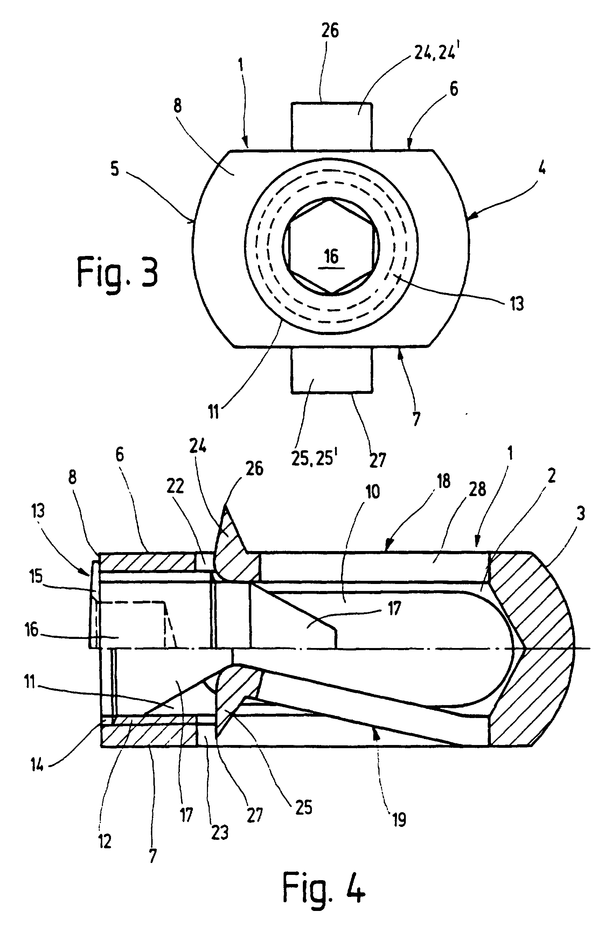

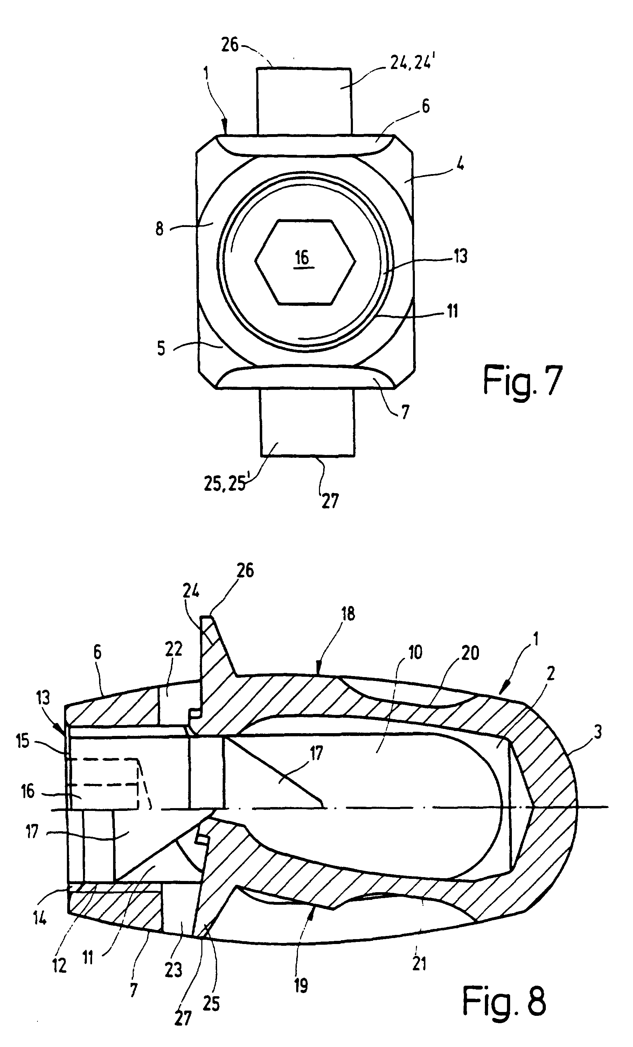

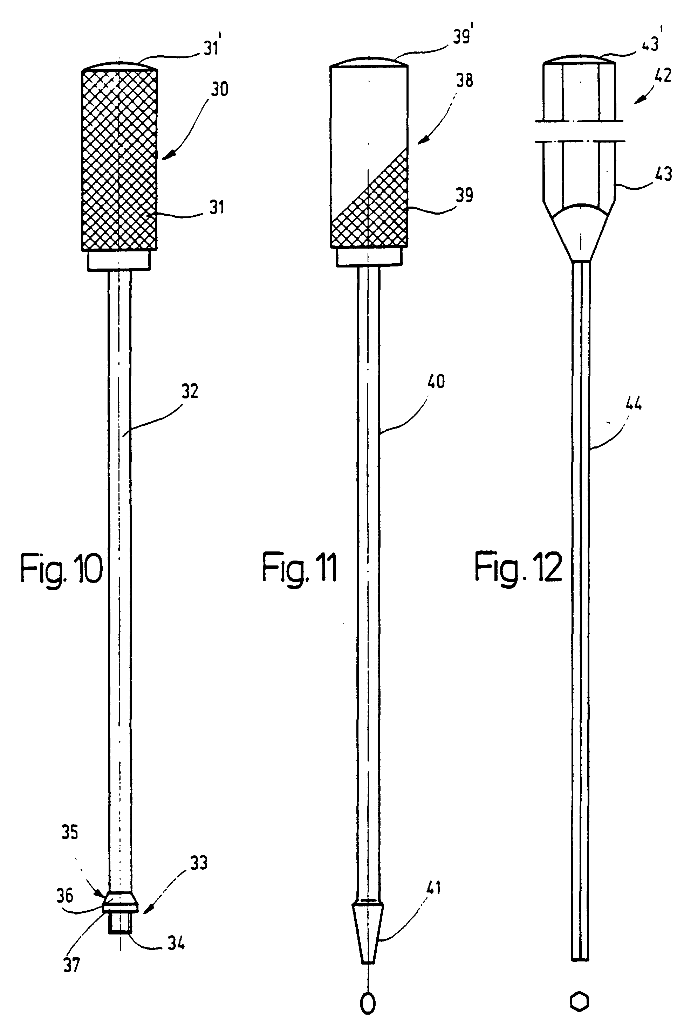

In den Figuren 4 bzw. 8 ist jeweils die Funktionsweise dieser Ausführungsbeispiele dargestellt. Die Figuren 10 bis 12 zeigen schematisch Ausführungsbeispiele der dazu benötigten Instrumente. Dies sind ein Implantathalter 30 (Figur 10), ein Distraktor (Figur 11) und ein Sechskant-Schraubendreher 42 (Figur 12). Allen Instrumenten 30, 38, 42 ist gemeinsam, daß sie einen Griff 31, 39, 43, einen langen Schaft 32, 40, 44 und ein Ende 33, 41, 45 aufweisen.

In dem Zustand (Figuren 4 bzw. 8 unten), in dem die Hohlkörper 1 zwischen Wirbelkörper eingesetzt werden (Einsetzzustand), sind die Aufspreizzungen 18, 19 in den Hohlraum 2 nach innen eingedrückt, so daß die freien Kanten 26, 27 der Krampen 24, 25 nicht über die Seitenflächen 6, 7 des Hohlkörpers 1 hinausragen. In das Innengewinde 12 der Bohrung 11 wird das vordere Ende 33 des Implantathalters 30, das mit einem Außengewinde 34 versehen ist, eingedreht. Das derart auf dem Implantathalter 30 fixierte und durch den Hohlkörper 1 gebildete Zwischenwirbelimplantat wird zwischen zwei Wirbelkörper eingetrieben. Wenn sich das Zwischenwirbelimplantat an der gewünschten Stelle befindet, wird das Ende 33 des Implantathalters 30 aus der Bohrung 11 des das Zwischenwirbelimplantat bildenden Hohlkörpers 1 herausgedreht. Nun werden mit Hilfe des Distraktors 38 (Figur 11), dessen Ende 41, wie angedeutet, etwa elliptischen Querschnitt aufweist, die Aufspreizzungen 18, 19 allmählich nach außen gedrückt und die scharf ausgebildeten Kanten 26, 27 der Krampen 24, 25 nach und nach in die Substantia Corticalis der Wirbelkörper eingedrückt. Dann wird mit Hilfe des Schraubendrehinstruments 42 die Innensechskant-Schraube 13 in das Innengewinde 12 der Bohrung 11 eingedreht (Figuren 4 bzw. 8 unten). Die Schraube 13 ist so lang, daß sie, im Querschnitt betrachtet, mit ihrem vorderen konisch sich verjüngenden Ende 17 über die Höhe der Krampen 24, 25 hinaus in den Hohlraum 2 des Hohlkörpers 1 hineinragt (Figuren 4 bzw. 8 oben) und somit beim Eindrehen die Aufspreizungen 18, 19 und mit ihnen die Krampen 24, 25 auseinander drückt. Die Dicke des Endes 17 entspricht etwa dem Durchmesser des Hohlraums 2, so daß beim Eindrehen die Aufspreizzungen 18, 19 festgestellt und die Lage des Hohlkörpers 1 zwischen den Wirbelkörpern fixiert wird.

Eine alternative Vorgehensweise beteht darin, die Aufspreizzungen 18, 19 mit Hilfe des Distraktors lediglich sanft nach außen zu gedrücken, bis die Kanten 26, 27 der Krampen 24, 25 an den jeweiligen Wirbelkörpern anliegen und nur ganz leicht in die Knochenhaut der Wirbelkörper eingedrückt sind. Erst beim Eindrehen der Schraube 13 werden die scharfen Kanten 26, 27 der Krampen 24 und 25 endgültig in die Substantia Corticalis der Wirbelkörper eingedrückt.

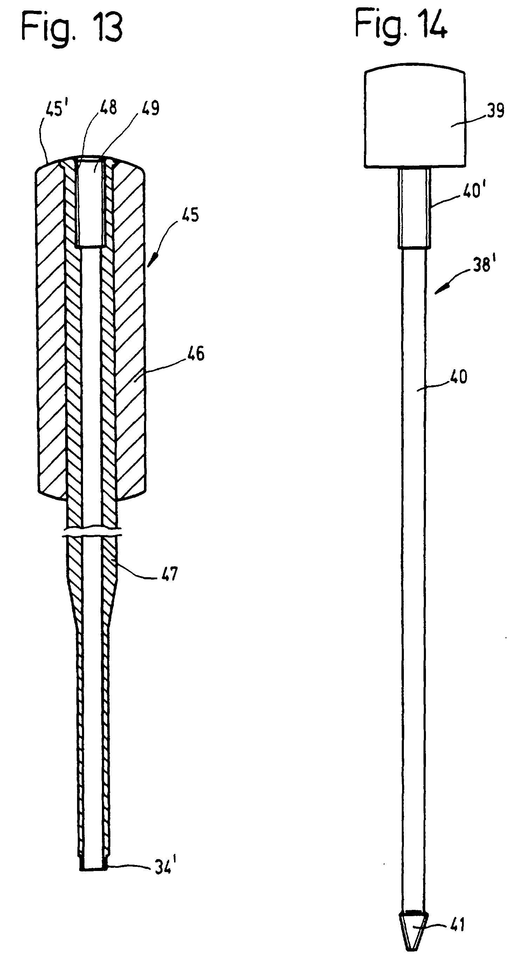

Figur 13 zeigt ein weiteres Ausführungsbeispiel eines Implantathalters 45 mit einem hohlen Griff 46, der vollständig von einem ebenfalls hohlen Schaft 47 durchsetzt ist. Das freie Ende des hohlen Schafts 47 weist ein Außengewinde 34 auf. Das griffseitige Ende des hohlen Schafts 47 besitzt ein Innengewinde 48. Die Prallfläche 45' des Griffs 46 weist eine Durchtrittsöffnung 49 auf, die den Zugang zu dem hohlen Schaft freigibt.

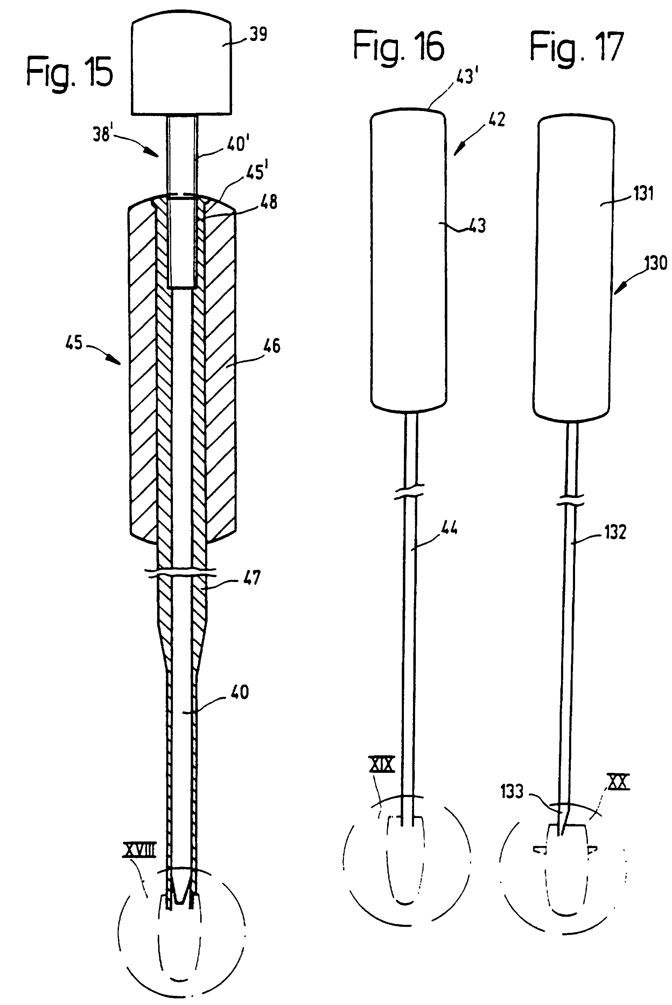

Die Funktion dieses Implantathalters 45 ist dieselbe, wie sie bereits für den in Figur 10 dargestellten Implantathalter 30 beschrieben wurde. Das vordere Ende des Implantathalters 45 wird mit seinem Außengewinde 34' in das Innengewinde 12 des Hohlkörpers 1 eingeschraubt. Auf diese Weise wird das Implantat gehalten, während es vom Operateur eingesetzt wird. Sitzt das Implantat an der richtigen Stelle, bleibt das vordere Ende des hohlen Schafts 46 mit dem Außengewinde 34 in das Innengewinde 12 der Bohrung 11 des Hohlkörpers 1 eingedreht. Der in Figur 14 dargestellte Distraktor 38', dessen Schaft 40 an seinem dem Griff 39 zugewandten Ende mit einem Außengewinde 40' versehen ist, wird in den hohlen Schaft 47 des Implantathalters 45 eingesteckt. Diese Situation ist in Figur 15 dargestellt. Das Gewinde 40' am griffseitigen Ende des Distraktors 38' greift in das Gewinde 48 am griffseitigen Ende des hohlen Schafts 47 ein. Durch Drehen des Distraktors 38' wird dieser mit dem Implantathalter 45 verschraubt. Es findet eine kontrollierte Verschiebung des Distraktors 38' statt, so daß seine Spitze 41 langsam in den Hohlraum 2 des Hohlkörpers 1 eindringt und die Aufspreizzungen 18, 19 aufweitet. Auf diese Weise kann das Zwischenwirbelimplantat kontrolliert geweitet werden, und man behält über den Implantathalter 45 gleichzeitig die volle Kontrolle über die Position und den Sitz des Implantats.

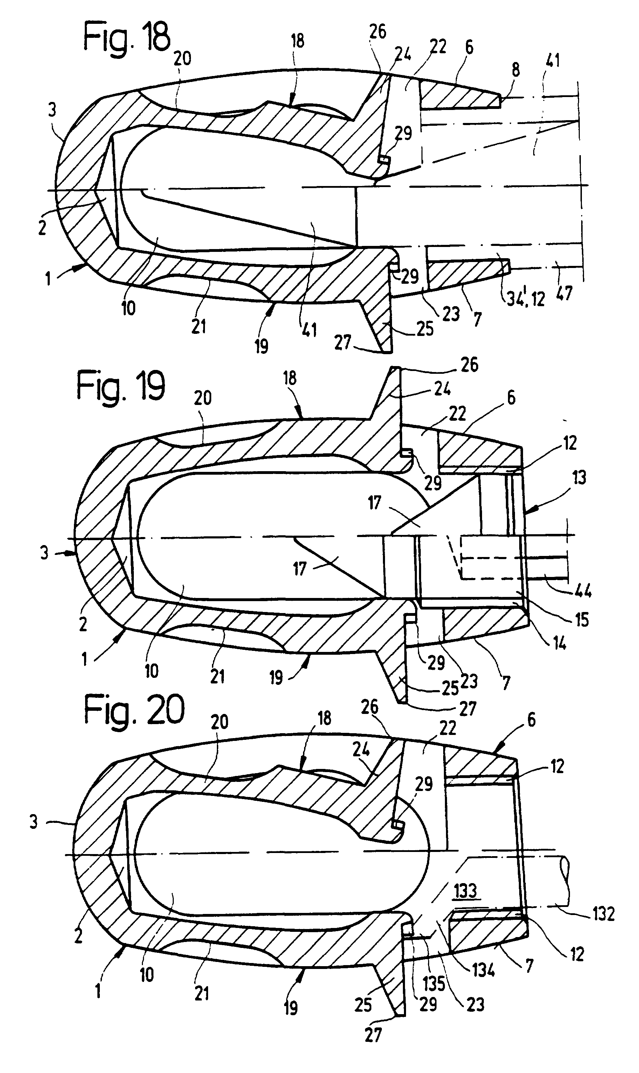

Anfangs- und Endstadium dieses Vorgangs sind in Figur 18 noch einmal vergrößert dargestellt, wobei der obere Teil der Figur die Situation zeigt, wie sie Figur 15 entspricht, nämlich nach dem Einsetzen und vor dem Einschrauben des Distraktors 38'. Der untere Teil der Figur 18 zeigt die Situation, nachdem der Distraktor 38' vollständig in den Implantathalter 45 eingeschraubt wurde. Das Zwischenwirbelimplantat ist jetzt vollständig geweitet. Selbstverständlich kann man auch in jedem Zwischenstadium die Aufweitung beenden und den Distraktor 38' aus dem Implantathalter 45 herausschrauben. Der gewünschte Endpunkt kann z.B. dadurch festgelegt werden, daß die Tiefe, in der die beiden Gewinde 47, 40' ineinander einschraubbar sind, entsprechend begrenzt ist.

Nun ist das Implantat "gesetzt". Der Distraktor 38' wird herausgeschraubt und aus dem Implantathalter 45 gezogen. Dann wird der Implantathalter aus dem Hohlkörper 1 herausgeschraubt. Die Schraube 13 wird auf einen Sechskant-Schraubendreher 42 (Figur 16) gesetzt, der dem in Figur 12 gezeigten Instrument entspricht und daher mit denselben Bezugszeichen versehen ist. Die Schraube 13 wird durch langsames Drehen des Schraubendrehinstruments 42 in den Hohlkörper 1 eingeschraubt. Diese Situation ist in Figur 19, oberer Teil, dargestellt. Wenn die Schraube 13 vollständig eingedreht ist, wird der Schraubendreher 42' abgesetzt. Damit ist die mit Hilfe des Distraktors 38' vorgenommene Aufspreizung der Zungen 18, 19 vollendet, und das Zwischenwirbelimplantat ist fixiert. Diese Situation ist in Figur 19 unten dargestellt. Natürlich kann man im ersten Schritt das Implantat nur teilweise aufweiten und die Aufweitung mit Hilfe der Schraube zu Ende führen, die einfach aus dem Implantathalter 30' herausgezogen wird.

In Figur 17 ist ein Rückholinstrument 130 dargestellt, das dann zum Einsatz kommt, wenn das Zwischenwirbelimplantat gemäß Figur 5 aus irgendeinem Grund explantiert werden muß.

Das Rückholinstrument 130 weist einen Griff 131, einen Schaft 132 und eine Spitze 133 auf. Während die Darstellung in Figur 17 rein schematisch ist, zeigt die Darstellung in Figur 20 detailliert die Konstruktion der Spitze 133 des Rückholinstruments 130. Die Spitze 133 ist im Ausführungsbeispiel derart angepaßt, daß sie nach Art einer Abkröpfung zweimal geknickt ist und einen Abschnitt 134 sowie einen mit dem Schaft 132 parallelen Abschnitt 135 aufweist. Dieser Knick ist so bemessen, daß nach dem Ausdrehen der Schraube 13 aus dem Hohlkörper 1 und dem Einsetzen des Rückholinstruments 130 der Abschnitt 135 der Spitze 133 in den Absatz 29 der Aufspreizzungen 18, 19 eingreift. Durch Abstützen des Rückholinstruments 130 an der Schulter des Gewindes 12 für die Schraube 13, kann die Aufspreizzunge 18, 19 wieder in den Hohlraum 2 zurückgebogen werden. Dies ist in Figur 20 oberer Teil dargestellt.

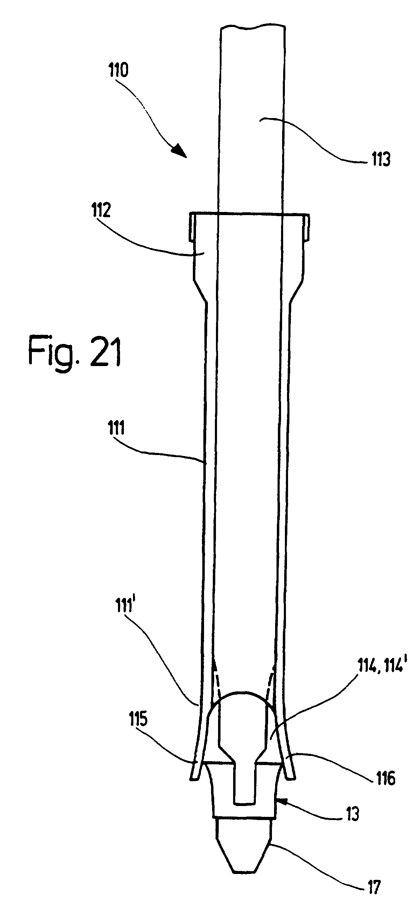

Figur 21 zeigt ein weiteres Ausführungsbeispiel eines Sechskant-Schraubendrehers 110 bestehend aus einer Hülse 111 mit Griffbereich 112 und dem Schaft 113 des eigentlichen Schraubendrehinstruments, der in der Hülse 111 geführt ist. Im vorderen Ende 111' der Hülse 111 befinden sich zwei U-förmige Einschnitte 114, 114'. Dadurch entstehen zwei Lappen 115, 116, die derart auseinanderspreizbar sind, daß dabei während des Einsetzens der Schraube 13 diese an ihrem Bund gehalten wird. Die Hülse 111 ist auf dem Schaft 113 des Schraubendrehinstruments verschiebbar. Ist die Schraube 13 eingesetzt, so verschiebt man die Hülse 111 auf dem Schaft 113 nach oben, so daß die Lappen 115, 116 die Schraube 13 freigeben.

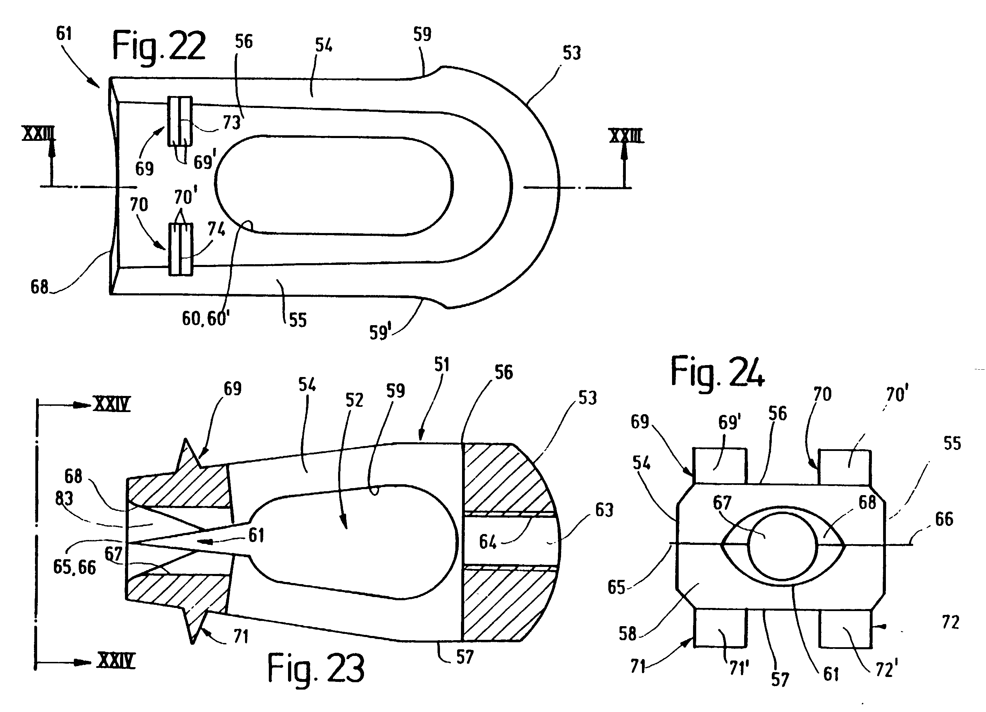

Ein drittes und viertes Ausführungsbeispiel eines erfindungsgemäßen Zwischenwirbelimplantats, die ebenfalls untereinander funktionsgleich sind, sind in den Figuren 22 bis 26 bzw. 27 bis 31 dargestellt. Gleiche Teile sind mit gleichen Bezugszeichen versehen. Es ist etwa 25 mm lang und hat einen Durchmesser von etwa 10 bis 14 mm. Dieses Ausführungsbeispiel weist einen Hohlraum 52, eine Kuppe 53, vier Seitenflächen 54, 55, 56, 57 und eine Rückfläche 58 auf. In alle Seitenflächen 54, 55, 56, 57 sind ovale Öffnungen 59, 59', 60, 60' eingelassen. Die Kuppe 53 weist eine Bohrung 63 mit einem Innengewinde 64 auf. In der Rückfläche 58 ist ferner eine Senkung 83 und Durchgangsöffnung 61 vorgesehen. Somit ergeben sich in der aus den Figuren ersichtlichen Weise ineinander übergehende Anlageflächen 67 und 68, wobei im zusammengedrückten Zustand des Hohlkörpers 5 die Anlagefläche 68 (gebildet durch die Senkung 83) einen größeren Durchmesser aufweist und sich konisch verjüngt und die Anlagefläche 67 (gebildet durch die Bohrung 61) einen kleineren Durchmesser aufweist und die eigentliche Durchgangsöffnung 61 bildet.

Die beiden einander gegenüberliegende Seitenflächen 56, 57 sind durch je einen Schlitz 65, 66 geteilt. Jeder Schlitz erstreckt sich von der Kante, an der die Rückfläche 58 in die entsprechenden Seitenfläche 56, 57 übergeht, bis zu den Öffnungen 60, 60' in den Seitenflächen 56, 57. Auf den dadurch entstehenden Hälften der Rückfläche 58 sind damit auch die Anlageflächen 67, 68 halbiert.

Außen an den Seitenflächen 54, 55, die parallel zu den Schlitzen 65, 66 verlaufen und nicht halbiert sind, sind in der Nähe des der Kuppe 53 gegenüberliegenden Endes je zwei Krampen 69, 70, 71, 72 einstückig angeformt. Auch diese Krampen sind im Querschnitt dreieckig und ihre freien Kanten 73, 74, 75, 76 stehen von den Seitenflächen 54, 55 weg etwa um 2 mm nach außen vor. Auch diese Kanten sind scharf ausgebilet, z.B. angeschliffen und bilden die erwähnten Fixierelemente.

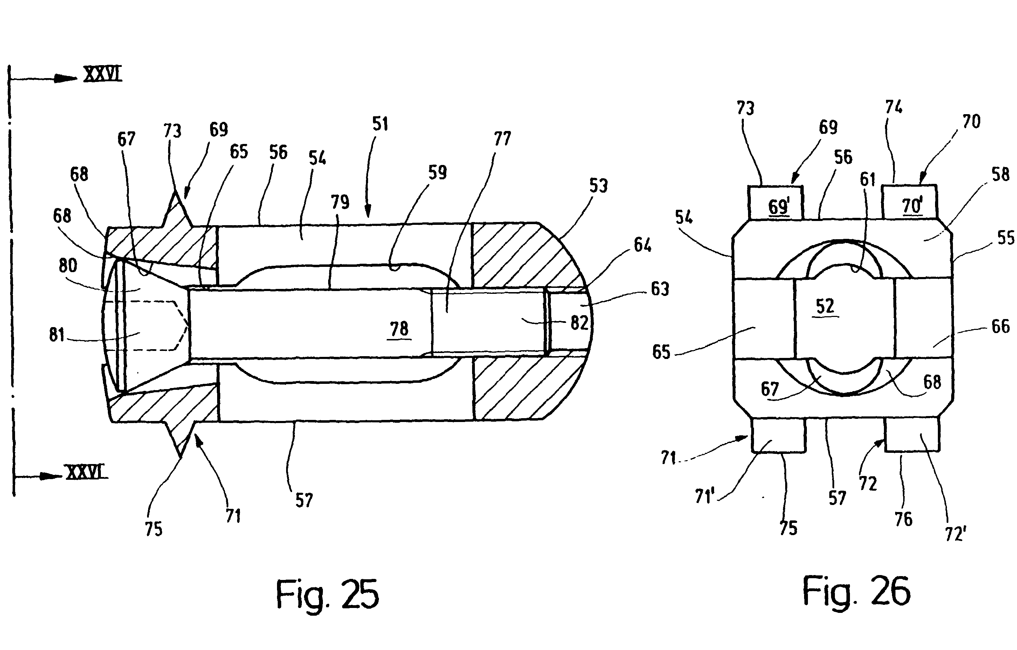

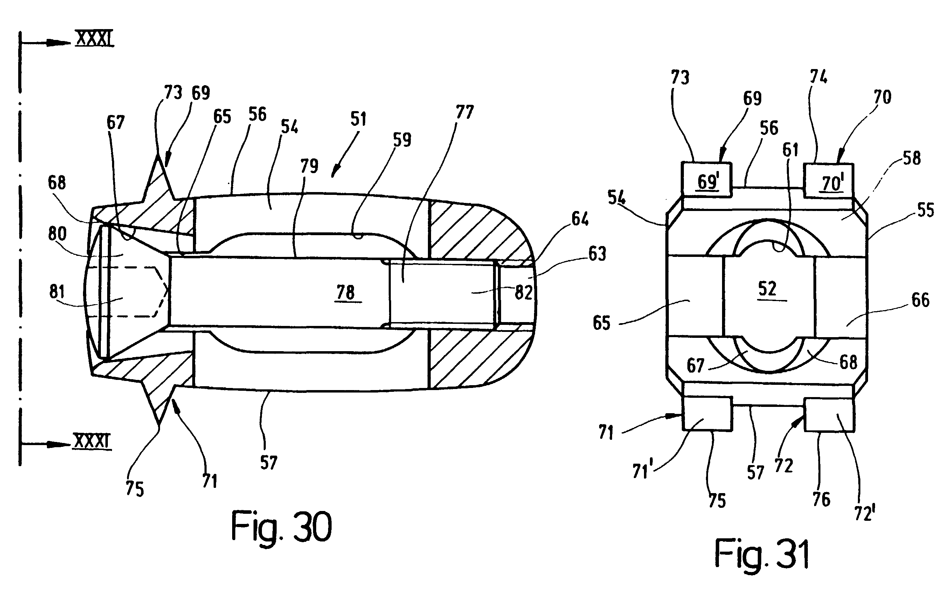

In den Figuren 25 und 30 ist eine Schraube 78 mit einem Schaft 79 und einem Außengewinde 77 gezeigt, deren Ende 82 in das Innengewinde 64 der Bohrung 63 eingeschraubt ist. Der Kopf 80 weist einen Innensechskant 81 auf und liegt mit seiner äußeren Umfangsfläche an der Anlagefläche 68 an.

Der Unterschied zwischen diesen Ausführungsbeispielen besteht ebenfalls darin, daß das dritte Ausführungsbeispiel (Figuren 22 bis 26) vier flache Seitenflächen 54 bis 57 aufweist, während das vierte Ausführungsbeispiel wieder eine insgesamt gewölbte, konvexe Kontur und ein "balliges" oder "bauchiges" Aussehen hat.

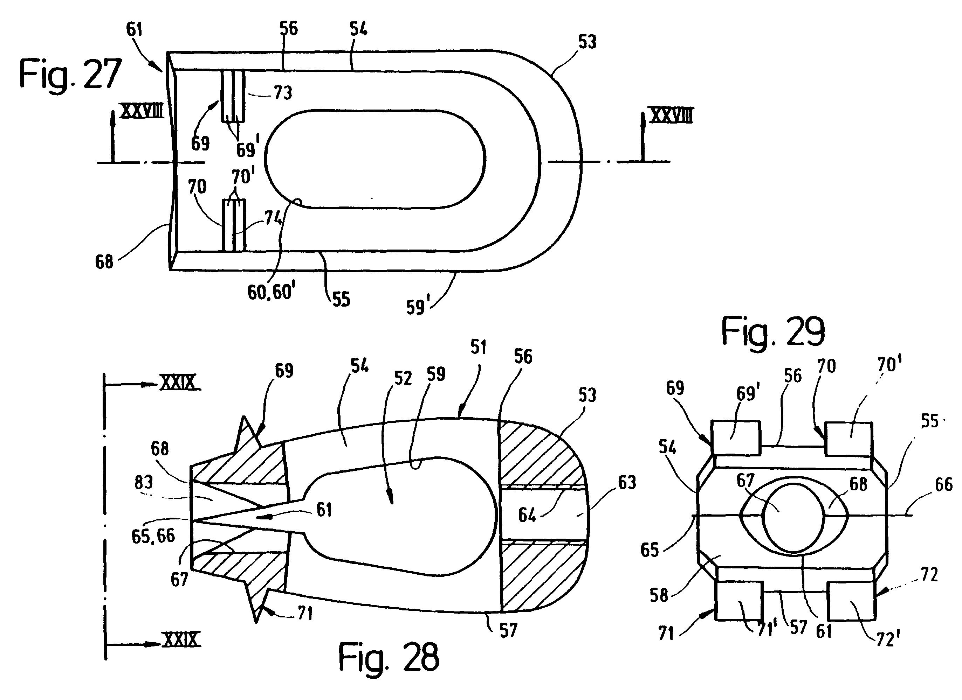

Die Funktionsweise des dritten und vierten Ausführungsbeispiels ist die folgende: Die Ausgangssituation ergibt sich aus den Figuren 22 und 23 bzw. 27 und 28. Die Seitenflächen 54, 55 sind zusammengepreßt, so daß die Kanten der Schlitze 65, 66 verengt zur Rückfläche 68 aneinanderanliegen (oder nur geringen Abstand haben) und die Krampen 69, 70, 71, 72 gegenüber einer gedachten Verlängerungslinie der parallen Abschnitte der Seitenflächen 56, 57 etwas zurückgezogen sind. Dies ist die Einsetzstellung. Die innere Anlagefläche 67 bildet, wie oben bereits erwähnt, in dieser Stellung einen kreiszylindrischen Durchgang (vgl. Figuren 23 und 28).

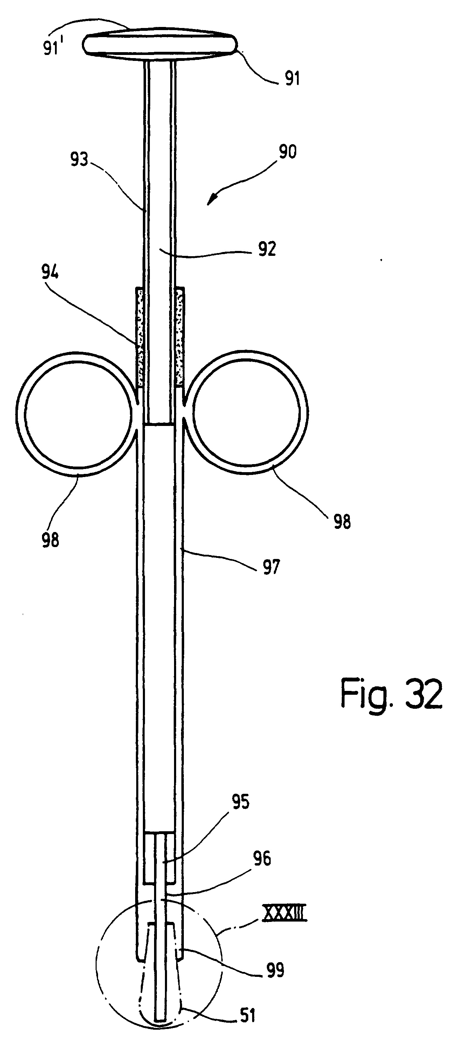

Zur Implantation dient der Impaktor 90 nach Figur 32. Er weist einen Griff 91 und einen mit einem Außengewinde 93 versehenen Schaft 92 auf. Auf dem Schaft 92 sitzt eine Stellmutter 94. Der Schaft 92 ist von einer Gewindespindel 95 durchsetzt, die an ihrem Ende mit einem Außengewinde 96 versehen ist. Die Gewindespindel kann mit dem Griff 91 gedreht werden. Sie dient als Implantathalter, d.h. sie wird in die Bohrung 63 an der Kuppe 53 des Hohlkörpers 51 eingeschraubt. Auf dem Schaft 92 ist eine Halterung 97 verschiebbar gehalten, die an ihrem oberen Ende zwei Griffe 98 aufweist, und an deren unteres Ende eine Hülse 99 angeformt ist, die den Hohlkörper 51 in dem Zustand, in dem er zwischen Wirbelkörper eingesetzt wird, umfaßt.

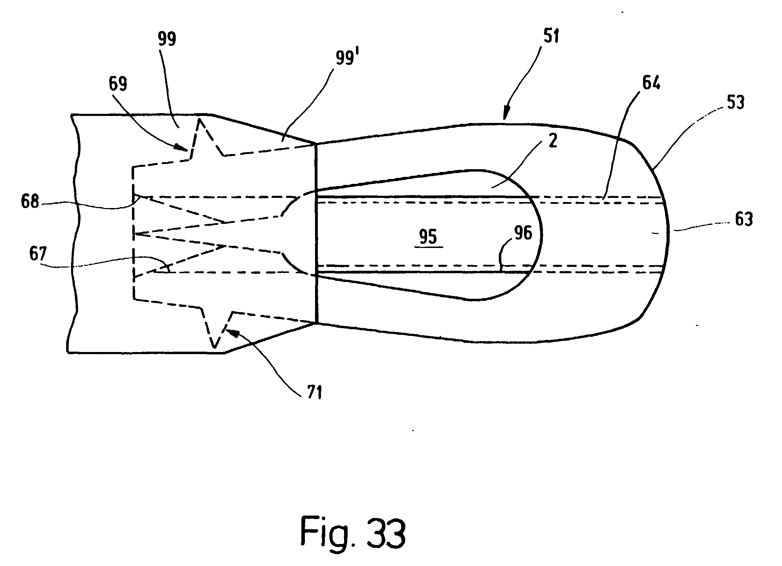

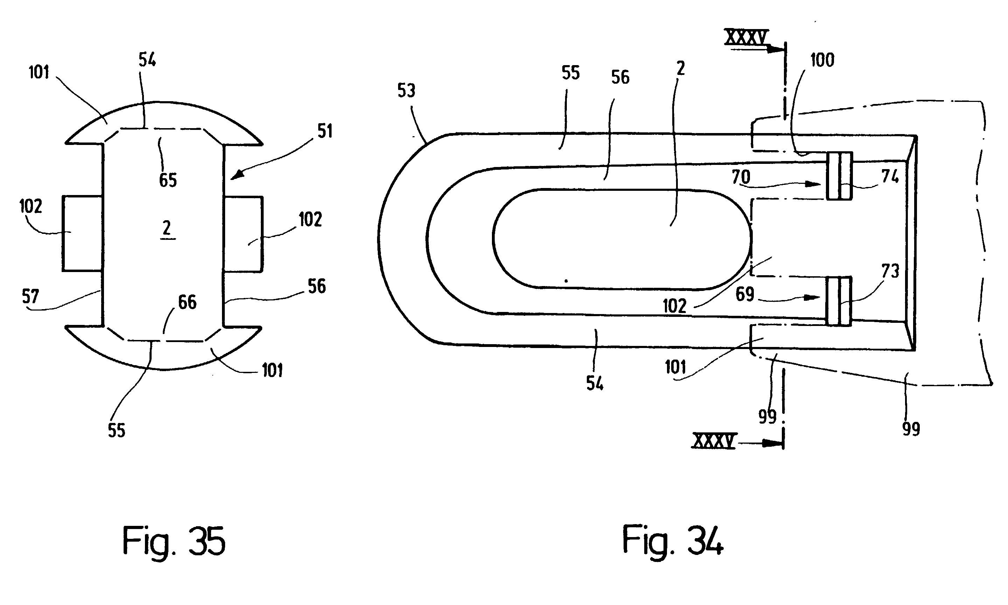

Die Art und Weise dieser Umfassung geht aus den Figuren 33 bis 35, in denen das vierte Ausführungsbeispiel gezeigt ist, hervor. Aus Figur 33 ist zu ersehen, daß die Hülse 99 die Krampen 69, 70, 71, 72 vollständig umschließt und zu ihrem Ende 99' hin abgeflacht ist, so daß ein glatter Übergang zwischen der Hülse 99 und dem Hohlkörper 51 gewährleistet ist. Die Gewindespindel 95 durchsetzt den Hohlraum 2 und ist mit ihrem Ende in die Bohrung 63 an der Kuppe 53 des Hohlkörpers 51 eingeschraubt. Figur 34 zeigt diesen Zustand in einer um 90° gedrehten Ansicht, wobei man sieht, daß das Ende 99' Ausnehmungen 100 aufweist, die die Krampen 69, 70, 71, 72 aufnehmen. Dadurch wird das Ende 99' der Hülse 99 in zwei breite Bereiche 101 und zwei schmale Bereiche 102 aufgeteilt, die den Hohlkörper 51 umfassen und in Einsetzstellung festhalten.

Das nunmehr so in seiner Einsetzstellung fixierte Zwischenwirbelimplantat (Hohlkörper 51) wird dann zwischen zwei Wirbelkörper eingetrieben, zum Beispiel eingeschlagen, wozu die Prallfläche 91' des Griffes 91 benötigt wird. Sowie das Zwischenwirbelimplantat positioniert ist, wird die Stellmutter 94 gelockert und die Halterung 97 an den Griffen 98 gefaßt und nach oben gezogen. Dann wird die Gewindespindel 95 aus der Bohrung 63 herausgeschraubt. Anschließend wird das Zwischenwirbelimplantat (Hohlkörper 1) geweitet.

Dies erfolgt derart, daß die Schraube 77 mit ihrem Innensechskant 81 auf das Ende des Schraubendreherinstruments 42 (siehe Figur 12) aufgesetzt wird. Die Schraube bildet das Aufweitelement. Durch die Feinabstimmung der Maße muß dafür gesorgt sein, daß die Schraube 77 durch eine sanfte Klemmung in dieser Position gehalten wird. Dann führt man mit Hilfe des Schraubendreherinstruments 42 die Schraube 77 in den Hohlkörper 51 ein und schraubt dann das Ende 82 in der Schraube 77 in das Innengewinde 64 der Bohrung 63, bis die äußere Umfangsfläche des Kopfes 80 durch Andruck an die Anlagefläche 68 die Seitenflächen 54 und 57 in die in Figur 9 gezeigte Stellung (oder mehr oder weniger weit) auseinandergedrückt hat, in der sich die Krampen 69 bis 72 in die Wirbelkörper eingegraben haben. Dann wird das Schraubendreherinstrument 42 abgezogen. Auf diese Weise kann man also variable Aufweitungen des Hohlkörpers 51, der das Zwischenwirbelimplantat ist, erreichen.

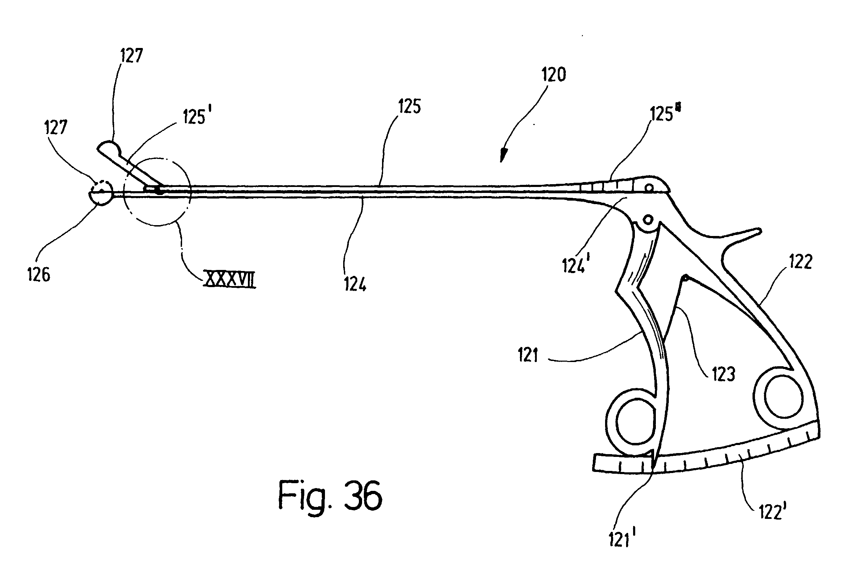

Figur 36 zeigt ein Meßinstrument 120 zur Messung des Abstands zwischen zwei Wirbelkörpern. Vor der Implantation eines Zwischenwirbelimplantats muß der Abstand zwischen zwei Wirbelkörpern gemessen werden, um die Größe des benötigten Implantats festlegen zu können (vgl. die Darstellung in Figur 9). Die Verstellbarkeit des erfindungsgemäßen Zwischenwirbelimplantats liefert einen Spielraum von einigen Millimetern. Dieser Spielraum reicht bei Patienten unterschiedlicher Körpergröße (z.B. Frauen und Männer) nicht aus. Daher muß in diesen Fällen eine Vorauswahl unter verschiedenen Größen des Zwischenwirbelimplantats getroffen werden.

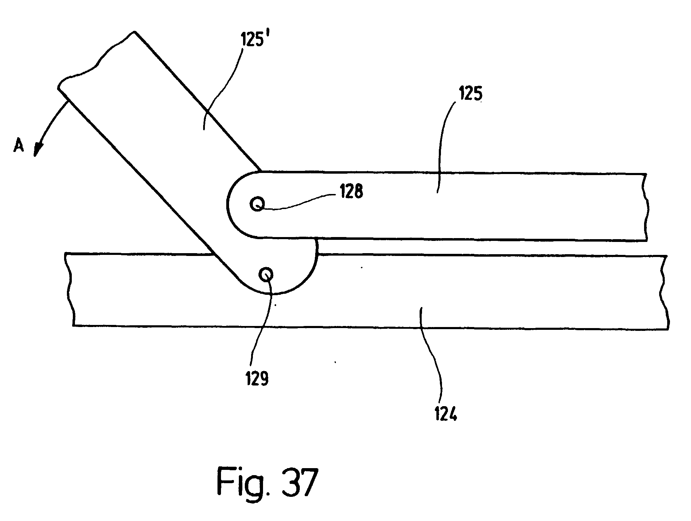

Das Meßinstrument 120 weist zwei Griffe 121, 122 auf, die von einer Feder 123 auseinandergehalten werden. Die beiden Griffe 121, 122 stehen in Verbindung mit je einem Schaft 124, 125. Der Schaft 125 ist an einem weiteren, kürzeren Schaft 125' angelenkt. Die Schafte 125, 125' haben zusammen dieselbe Länge wie der Schaft 124. Die Schafte 124 und 125' enden in zwei halbkugeligen Teilen 126, 127. Diese Teile 126, 127 dienen als Meßfühler. In dem in Figur 36 gezeigten entspannten Zustand sind die halbkugeligen Teile 126, 127 aufgeweitet. Durch Zusammenführen der Griff 121, 122 nähern sich die Halbkugeln 126, 127 einander an, wie es in Figur 36 gestrichelt dargestellt ist. Die mechanische Verbindung, die diese Zusammenführung bewirkt, ist in Figur 37 noch einmal deutlicher dargestellt. Durch das Zusammendrücken der Griffe 121, 122 bewegt sich der Schaft 125 nach vorn, d.h. in Richtung der Halbkugel 126. Er nimmt den Schaft 125', der in die Halbkugel 127 ausläuft, mit sich. Dabei dreht sich der Schaft 125' im Gelenk 128. Da der Schaft 125' aber auch an dem Gelenk 129 am Schaft 124 angelenkt ist, bewegt er sich nicht mit dem Schaft 125 nach vorne, sondern wird nach unten in Richtung des Pfeils A bewegt.

Mit den halbkugeligen Teilen 126, 127 fährt man in den Zwischenraum zwischen zwei Wirbelkörper und weitet diese dann durch Entspannen der Griffe 121, 122 wieder auf. Der gemessene Abstand kann auf mehrere Arten und Weisen abgelesen werden. Zwei davon sind in Figur 36 angedeutet. Am Griff 122 kann ein Streifen 122' mit einer Skalierung befestigt sein, während der Griff 121 mit einem Zeiger 121' versehen ist. Je größer der gemessene Abstand ist, desto größer wird der Abstand zwischen dem Griff 121 und dem Griff 122. Dieser Abstand ist auf der Skala ablesbar. Eine andere Möglichkeit ist eine Skala 125" am Ende des Schaftes 125. In diesem Fall ist das Maß der Vorwärtsbewegung des Schaftes 125 gegenüber dem Schaft 124 ein Maß für den gemessenen Abstand zwischen den Wirbelkörpern. Der zurückgelegte Weg kann dann an der Skala 125" über einen auf dem Schaft 124 festmarkierten Punkt 124' abgelesen werden.

## Patentansprüche

1. Zwischenwirbelimplantat mit einem etwa zylinderförmigen Hohlkörper (1, 51), der zwei Enden (3, 8, 53, 58), vier Seitenflächen (4, 5, 6, 7, 54, 55, 56, 57) sowie eine oder mehrere Öffnungen (9, 10, 59, 59', 60, 60') aufweist und mit zwei einander gegenüberliegenden aufspreizbaren Bereichen (18, 19; 56, 57) versehen ist, die zur Fixierung von Wirbelkörpern Fixierelemente (24, 25, 69 - 72) aufweisen, wobei die aufspreizbaren Bereiche an ihren einen Enden miteinander verbunden und an ihren anderen freien Enden (26, 27) durch ein Aufweitelement (13) aufspreizbar sind, und bei dem ein Ende (8, 53) des Zwischenwirbelimplantats eine mit einem Innengewinde (11, 64) versehene Öffnung (12, 63) aufweist, **dadurch gekennzeichnet, dass** das Aufweitelement eine Schraube (13, 78) ist, die in die mit dem Innengewinde (11, 64) versehene Öffnung (12, 63) einschraubbar ist, und dass die Schraube (13, 78) mit ihrem Ende (17) oder ihrem Kopf (80) beim Einschrauben die freien Enden der aufspreizbaren Bereiche (18, 19; 56, 57) auseinanderdrückt.

2. Zwischenwirbelimplantat nach Anspruch 1, **dadurch gekennzeichnet, dass** die aufspreizbaren Bereiche (18, 19) in den einander gegenüberliegenden Seitenflächen (6, 7) eines etwa zylindrischen Hohlkörpers (1) angeordnet sind.

3. Zwischenwirbelimplantat nach Anspruch 2, **dadurch gekennzeichnet, dass** die mit dem Innengewinde (11) versehene Öffnung (12) in einer Rückfläche (8) des Hohlkörpers (1) angeordnet ist.

4. Zwischenwirbelimplantat nach Anspruch 3, **dadurch gekennzeichnet, dass** die aufspreizbaren Bereiche (18, 19) über eine der Rückfläche (8) gegenüberliegende Kuppe (3) des Hohlkörpers (1) miteinander verbunden und mit dieser (3) einteilig ausgebildet sind.

5. Zwischenwirbelimplantat nach Anspruch 4, **dadurch gekennzeichnet, dass** die aufspreizbaren Bereiche als Zungen (18, 19) in die einander gegenüberliegenden Seitenflächen (6, 7) des Höhlkörpers (1) eingeformt sind.

6. Zwischenwirbelimplantat nach Anspruch 5, **dadurch gekennzeichnet, dass** die Zungen (18, 19) in die Seitenflächen durch U-förmige Schlitze (22, 23) eingeformt sind.

7. Zwischenwirbelimplantat nach Anspruch 3, **dadurch gekennzeichnet, dass** die aufspreizbaren Bereiche (18, 19) je einen Absatz (29) aufweisen, in die durch die genannte Öffnung (12) hindurch ein Rückholinstrument (130) einsetzbar ist, um die aufgeweiteten Bereiche (18, 19) in ihre Ausgangslage zurückzubiegen.

8. Zwischenwirbelimplantat nach Anspruch 7, **dadurch gekennzeichnet, dass** die Absätze (29) an den freien Enden der aufspreizbaren Bereiche (18, 19) angebracht sind.

9. Zwischenwirbelimplantat nach Anspruch 7 oder 8, **dadurch gekennzeichnet, dass** die Absätze (29) rund sind.

10. Zwischenwirbelimplantat nach Anspruch 3, **dadurch gekennzeichnet, dass** die Schraube (13), die die aufspreizbaren Bereiche (18, 19) auseinander drückt, ein sich konisch verjüngendes Ende (17) aufweist.

11. Zwischenwirbelimplantat nach Anspruch 1, **dadurch gekennzeichnet, dass** es eine konvexe Kontur aufweist.

12. Zwischenwirbelimplantat nach Anspruch 1, **dadurch gekennzeichnet, dass** die aufspreizbaren Bereiche (56, 57) Seitenflächen (54, 55) aufweisen, die von einer sie verbindenden Kuppe (53) her durch Schlitze (65, 66) getrennt sind, dass die mit dem Innengewinde (64) versehene Öffnung (63) zur Aufnahme der Schraube (78) in der Kuppe (53) angeordnet ist, und dass die freien Enden der aufspreizbaren Bereiche (56, 57) im nicht aufgeweiteten Zustand zwischen sich einen Durchgang (67) bilden, durch den hindurch die Schraube (78) in die Öffnung (63) einschraubbar ist, und dass Teilflächen (68) des Durchgangs beim Einschrauben der Schraube (78) auseinander gedrückt werden.

13. Zwischenwirbelimplantat nach Anspruch 12, **dadurch gekennzeichnet, dass** sich die Schraube (78) und/oder die mit ihr zusammenwirkenden Fläche (67, 68) in Längsrichtung konisch verjüngen.

14. Zwischenwirbelimplantat nach Anspruch 1 oder einem der folgenden, **dadurch gekennzeichnet, dass** die mit dem Innengewinde (11, 64) versehene Öffnung zur Aufnahme eines an seinem Ende mit einem Außengewinde (34, 95) versehenen Ende eines Implantathalters (30, 90) bestimmt ist.

15. Kombination eines Zwischenwirbelimplantates nach einem der Ansprüche 1 bis 11 mit einem Instrument (45) zum Einsetzen und Aufweiten des Zwischenwirbelimplantates, **dadurch gekennzeichnet, dass** in einem Griff (46) des Instrumentes (45) ein erster hohler Schaft (47) angeordnet ist, der an seinem griffseitigen Ende ein Innengewinde (48) und an seinem freien Ende ein Außengewinde (34') zum Einschrauben in die Öffnung (15) des Zwischenwirbelimplantates aufweist, und dass in dem ersten Schaft ein zweiter Schaft (40) einsetzbar ist, der an seinem griffnahen Ende ein in das genannte Innengewinde eingreifendes Außengewinde aufweist.

16. Kombination nach Anspruch 15, **dadurch gekennzeichnet, dass** der Griff (46) eine Prallfläche (45') aufweist.

17. Kombination eines Zwischenwirbelimplantates nach einem der Ansprüche 12 bis 14 mit einem Implantathalter (90) zum Halten und Einsetzen des Zwischenwirbelimplantats, **dadurch gekennzeichnet, dass** der Implantathalter (90) einen ersten hohlen Schaft (97) aufweist, der auf seiner Unterseite mit einer Aufnahmehülse (99) zur Aufnahme des Zwischenwirbelimplantates (51) versehen ist und einen in dem hohlen Schaft (97) einsetzbaren weiteren Schaft (92) aufweist, der auf dem erstgenannten Schaft (97) durch eine Stellmutter (94) gesichert ist, und dass eine mit einem Gewinde versehenen Stange (95) sich durch den zweitgenannten Schaft (92) hindurch erstreckt, deren vorderes Ende in die genannte Öffnung des Zwischenwirbelimplantats (51) einschraubbar ist.

18. Kombination nach Anspruch 17, **dadurch gekennzeichnet, dass** die Aufnahmehülse (99) weitere Ausnehmungen (100) zur Aufnahme der Fixierelemente des Zwischenwirbelimplantats aufweist.

19. Kombination nach Anspruch 18, **dadurch gekennzeichnet, dass** der Griff (91) des zweitgenannten Schaftes (92) eine Prallfläche (91') aufweist.

## Claims

1. An intervertebral implant comprising a substantially cylindrical hollow body (1, 51) which has two ends (3, 8, 53, 58), four side surfaces (4, 5, 6, 7, 54, 55, 56, 57) and one or more openings (9, 10, 59, 59', 60, 60'), and two mutually oppositely disposed spreadable regions (18, 19; 56, 57) having fixing elements (24, 25, 69-72) for fixing vertebrae, wherein the spreadable regions are connected together at their one ends and at their other free ends (26, 27) can be spread open by an expansion element (13), and in which one end (8, 53) of the intervertebral implant has an opening (12, 63) provided with a female screwthread (11, 64), **characterised in that** the expansion element is a screw (13, 78) which can be screwed into the opening (12, 63) provided with the female screwthread (11, 64) and that upon being screwed in the screw (13, 78) with its end (17) or its head (80) urges the free ends of the spreadable regions (18, 19; 56, 57) away from each other.

2. An intervertebral implant according to claim 1 **characterised in that** the spreadable regions (18, 19) are arranged in the mutually oppositely disposed side surfaces (6, 7) of a substantially cylindrical hollow body (1).

3. An intervertebral implant according to claim 2 **characterised in that** the opening (12) provided with the female screwthread (11) is arranged in a rear surface (8) of the hollow body (1).

4. An intervertebral implant according to claim 3 **characterised in that** the spreadable regions (18, 19) are connected together by way of a tip (3) of the hollow body (1), which is in opposite relationship to the rear surface (8), and are formed in one piece with said tip (3).

5. An intervertebral implant according to claim 4 **characterised in that** the spreadable regions are in the form of tongues (18, 19) formed in the mutually oppositely disposed side surfaces (6, 7) of the hollow body (1).

6. An intervertebral implant according to claim 5 **characterised in that** the tongues (18, 19) are formed in the side surfaces by way of U-shaped slots (22, 23).

7. An intervertebral implant according to claim 3 **characterised in that** the spreadable regions (18, 19) each have a respective step (29), into which steps a recovery instrument (130) can be inserted through said opening (12) in order to bend the expanded regions (18, 19) back into their starting position.

8. An intervertebral implant according to claim 7 **characterised in that** the steps (29) are disposed at the free ends of the spreadable regions (18, 19).

9. An intervertebral implant according to claim 7 or claim 8 **characterised in that** the steps (29) are round.

10. An intervertebral implant according to claim 3 **characterised in that** the screw (13) which urges the spreadable regions (18, 19) away from each other has an end (17) which tapers inwardly.

11. An intervertebral implant according to claim 1 **characterised in that** it is of a convex contour.

12. An intervertebral implant according to claim 1 **characterised in that** the spreadable regions (56, 57) have side surfaces (54, 55) which are separated from a tip (53) connecting them by slots (65, 66), that the opening (63) provided with the female screwthread (64) for receiving the screw (78) is arranged in the tip (53), and that the free ends of the spreadable regions (56), in the non-expanded condition, form between them a passage (67) through which the screw (78) can be screwed into the opening (63), and that surface portions (68) of the passage are urged away from each other when the screw (78) is screwed in.

13. An intervertebral implant according to claim 12 **characterised in that** the screw and/or the surface (67, 68) co-operating therewith taper inwardly in the longitudinal direction.

14. An intervertebral implant according to claim 1 or one of the following claims **characterised in that** the opening provided with the female screwthread (11, 64) is intended to receive an end of an implant holder (30, 90), provided at its end with a male screwthread (34, 95).

15. A combination of an intervertebral implant according to one of claims 1 to 11 with an instrument (45) for inserting and expanding the intervertebral implant, **characterised in that** arranged in a handle (46) of the instrument (45) is a first hollow shank (47) which at its handle end has a female screwthread (48) and at its free end a male screwthread (34') for screwing into the opening (15) of the intervertebral implant, and that a second shank (40) can be fitted into the first shank, said second shank having at its end near the handle a male screwthread which engages into said female screwthread.

16. A combination according to claim 15 **characterised in that** the handle (46) has an impact surface (45').

17. A combination of an intervertebral implant according to one of claims 12 to 14 comprising an implant holder (90) for holding and inserting the intervertebral implant, **characterised in that** the implant holder (90) has a first hollow shank (97) which is provided on its underside with a receiving sleeve (99) for receiving the intervertebral implant (51) and which has a further shank (92) which can be fitted in the hollow shank (97) and which is secured on the first-mentioned shank (97) by a setting nut (94), and that a bar (95) provided with a screwthread extends through the second-mentioned shank (92), the front end of which bar can be screwed into said opening in the intervertebral implant (51).

18. A combination according to claim 17 **characterised in that** the receiving sleeve (99) has further apertures (100) for receiving the fixing elements of the intervertebral implant.

19. A combination according to claim 18 **characterised in that** the handle (91) of the second-mentioned shank (92) has an impact surface (91').

## Revendications

1. Implant de conjugaison avec un corps creux (1, 51) à peu près cylindrique, qui présente deux extrémités (3, 8, 53, 58), quatre faces latérales (4, 5, 6, 7, 54, 55, 56, 57) et une ou plusieurs ouvertures (9, 10, 59, 59', 60, 60') et est pourvue de deux zones (18, 19 ; 56, 57) qui peuvent être écartées, se font face et présentent des éléments de fixation (24, 25, 69 - 72) pour la fixation de corps vertébraux, les zones écartables étant reliées entre elles sur leurs deux extrémités et pouvant être écartées par un élément d'élargissement (13) sur leurs autres extrémités (26, 27) libres, et sur lequel une extrémité (8 ; 53) de l'implant de conjugaison présente une ouverture (12, 63) pourvue d'un filetage intérieur (11, 64), **caractérisé en ce que** l'élément d'élargissement est une vis (13, 78) qui peut être vissée dans l'ouverture (12, 63) pourvue du filetage intérieur (11, 64), et **en ce que** la vis (13, 78) écarte les extrémités libres des zones (18, 19 ; 56, 57) écartables avec son extrémité (17) ou sa tête (80) lors du vissage.

2. Implant de conjugaison selon la revendication 1, **caractérisé en ce que** les zones (18, 19) écartables sont disposées dans les faces latérales (6, 7) opposées d'un corps creux (1) à peu près cylindrique.

3. Implant de conjugaison selon la revendication 2, **caractérisé en ce que** l'ouverture (12) pourvue du filetage intérieur (11) est disposée dans une face arrière (8) du corps creux (1).

4. Implant de conjugaison selon la revendication 3, **caractérisé en ce que** les zones écartables (18, 19) sont reliées entre elles par un sommet arrondi (3), opposé à la face arrière (8), du corps creux (1) et sont conçues d'un seul bloc avec celui-ci (3).

5. Implant de conjugaison selon la revendication 4, **caractérisé en ce que** les zones écartables sont réalisées sous la forme de languettes (18, 19) dans les faces latérales (6, 7) se faisant face du corps creux (1).

6. Implant de conjugaison selon la revendication 5, **caractérisé en ce que** les languettes (18, 19) sont réalisées dans les faces latérales à travers des fentes (22, 23) en forme de U.

7. Implant de conjugaison selon la revendication 3, **caractérisé en ce que** les zones (18, 19) écartables présentent chacune un décrochement (29), dans lequel on peut insérer un instrument de rappel (130) à travers l'ouverture (12) citée, afin de recourber les zones (18, 19) élargies dans leur position de départ.

8. Implant de conjugaison selon la revendication 7, **caractérisé en ce que** les décrochements (29) sont disposés sur les extrémités libres des zones (18, 19) écartables.

9. Implant de conjugaison selon la revendication 7 ou 8, **caractérisé en ce que** les décrochements (29) sont ronds.

10. Implant de conjugaison selon la revendication 3, **caractérisé en ce que** la vis (13), qui écarte les zones (18, 19) écartables, présente une extrémité (17) qui se rétrécit à la façon d'un cône.

11. Implant de conjugaison selon la revendication 1, **caractérisé en ce qu'**il présente un contour convexe.

12. Implant de conjugaison selon la revendication 1, **caractérisé en ce que** les zones (56, 57) écartables présentent des faces latérales (54, 55), qui sont séparées d'un sommet arrondi (53) qui les relie par des fentes (65, 66), **en ce que** l'ouverture (63) pourvue du filetage intérieur (64) pour le logement de la vis est disposée dans le sommet arrondi (53) et **en ce que** les extrémités libres des zones (56, 57) écartables forment dans l'état non élargi un passage (67) entre elles, par lequel la vis (78) peut être vissée dans l'ouverture (63), et **en ce que** des surfaces partielles (68) du passage sont écartées lors du vissage de la vis (78).

13. Implant de conjugaison selon la revendication 12, **caractérisé en ce que** la vis (78) et/ou la surface (67, 68) qui agit de concert avec elle se rétrécissent dans le sens longitudinal à la façon d'un cône.

14. Implant de conjugaison selon l revendication 1 ou l'une quelconque des revendications suivantes, **caractérisé en ce que** l'ouverture pourvue du filetage interne (11, 64) est destiné à loger une extrémité, pourvue sur son extrémité d'un filetage externe (34, 95), d'un support d'implant (30, 90).

15. Combinaison d'un implant de conjugaison selon l'une quelconque des revendications 1 à 11 avec un instrument (45) pour la mise en place et l'élargissement de l'implant de conjugaison, **caractérisé en ce que**, dans une poignée (46) de l'instrument (45), est disposée une première tige (47) creuse qui présente un filetage interne (48) sur son extrémité côté poignée et sur son extrémité libre un filetage externe (34') pour le vissage dans l'ouverture (15) de l'implant de conjugaison, et **en ce que**, dans la première tige, on peut insérer une deuxième tige (40) qui présente sur son extrémité proche de la poignée un filetage externe qui s'engage dans le filetage interne cité.

16. Combinaison selon la revendication 15, **caractérisé en ce que** la poignée (46) présente une surface de rebondissement (45').

17. Combinaison d'un implant de conjugaison selon l'une quelconque des revendications 12 à 14 avec un support d'implant (90) pour le maintien et la mise en place de l'implant de conjugaison, **caractérisée en ce que** le support d'implant (90) présente une première tige (97) creuse, qui est pourvue sur sa face inférieure d'une douille de logement (99) pour le logement de l'implant de conjugaison (51) et présente une autre tige (92) insérable dans la tige (97) creuse, qui est bloquée sur la première tige (97) citée par un écrou de serrage (94), et **en ce qu'**une barre (95) pourvue d'un filetage s'étend à travers la deuxième tige (92) citée, dont l'extrémité supérieure peut être vissée dans l'ouverture citée de l'implant de conjugaison (51).

18. Combinaison selon la revendication 17, **caractérisée en ce que** la douille de logement (99) présente d'autres évidements (100) pour le logement des éléments de fixation de l'implant de conjugaison.

19. Combinaison selon la revendication 18, **caractérisée en ce que** la poignée (91) de la deuxième tige (92) citée présente une surface de rebondissement (91').
